# FASCICULE DE BREVET EUROPEEN

(11) **EP 0 522 897 B1**
(45) Date de publication et mention de la délivrance du brevet: **08.11.1995**
(21) Numéro de dépôt: 92401588.6
(22) Date de dépôt: 09.06.1992
(51) Int. Cl.: G01N 33/10, G01N 1/20, G01F 19/00

(54) **Cellule de mesure pour produits granuleux ou pulvérulents**
Messzelle für Granulate oder Pulver
Measuring cell for granular or pulverulent products

(30) Priorité: 13.06.1991 FR 9107217
(43) Date de publication de la demande: 13.01.1993
(73) Titulaire: TRIPETTE & RENAUD Société Anonyme:, F-92396 Villeneuve La Garenne Cédex (FR)
(72) Inventeur: Le Gigan, Dominique, F-95620 Parmain (FR)
(74) Mandataire: Chambon, Gérard

(56) Documents cités:
- WO-A-89/10548
- FR-A- 778 157
- FR-A- 2 558 282
- PATENT ABSTRACTS OF JAPAN, vol. 6, no. 30 (P-103)(908), 23 février 1982/

## Description

L'invention concerne une cellule de mesure pour produits granuleux ou pulvérulents, par exemple destinée à équiper un appareil de mesure des teneurs de certaines substances dans le produit, et plus particulièrement encore la teneur en humidité dans les céréales ou similaires.

Il existe divers appareils de mesure concernant notamment les céréales en grains, et selon le type de mesure, il est souvent nécessaire de pouvoir reproduire des volumes répétitifs du produit. C'est le cas notamment lorsque l'on désire mesurer le poids spécifique du grain, ou lorsque la mesure peut dépendre du volume de l'échantillon, comme par exemple dans la mesure de l'humidité dudit grain, dans le cas d'une mesure à volume constant.

Or, pour remplir de manière répétitive et constante une cellule qui se présente sous forme d'un récipient, avec un produit granuleux ou pulvérulent, il est nécessaire de mettre à niveau ou sensiblement à niveau le produit à l'endroit de l'embouchure de la cellule, ledit produit formant en effet généralement un dôme. L'importance de ce dôme peut varier en fonction notamment des caractéristiques du produit ce qui influence bien sûr la valeur du volume.

C'est pourquoi on a déjà imaginé des systèmes à bras de balayage pour éliminer le dôme ainsi formé, comme décrit notamment dans le brevet FR-2389179 (ou le brevet US correspondant 4,193,116). De tels systèmes nécessitent des moyens mécaniques en mouvement et sont donc onéreux avec les risques de pannes inhérents.

Dans un système du type décrit dans le brevet JP-A-56150329, les mesures sont effectuées sur un volume limité de la cellule de telle sorte que ledit volume est connu dès que ce niveau est atteint et quel que soit le surplus de produit.

Pour palier certains inconvénients de l'art connu, l'inventeur a imaginé une cellule à auto-arasage, par simple effet gravitaire.

Une cellule selon l'invention est en effet remarquable en ce que le plan de son embouchure de remplissage, en position de fonctionnement, forme un angle dièdre aigu avec un plan horizontal afin de limiter par effet gravitaire l'importance du dôme formé par le produit après remplissage complet de la cellule, la mesure s'effectuant sur la quasi-totalité du produit ainsi contenu.

L'expérience montre qu'il peut paraître préférable que l'angle dudit dièdre soit inférieur ou égal à 45°.

Avantageusement, l'embouchure de remplissage de la cellule s'ouvre sur toute la surface de la partie supérieure de ladite cellule.

De préférence, la section horizontale de la cellule en position de fonctionnement est au moins en partie décroissante en partant du fond de ladite cellule vers son embouchure.

Selon un mode de réalisation, elle comporte une embouchure rectangulaire, deux parois latérales planes et parallèles raccordées sur les deux côtés opposés et inclinés de l'embouchure et deux autres parois raccordées sur les deux autres côtés horizontaux de ladite embouchure, toutes ces parois se prolongeant en outre à l'opposé de l'embouchure jusqu'au fond de la cellule.

De préférence dans ce cas, au moins l'une des parois, raccordée sur l'un des côtés horizontaux de l'embouchure, et qui part du fond de la cellule, est au moins en partie inclinée vers la paroi opposée qui est, elle, raccordée sur l'autre côté horizontal de l'embouchure. Au lieu d'être inclinée, ladite paroi peut avantageusement être incurvée.

En outre, il est clair que si l'une des deux parois susmentionnées peut être inclinée ou incurvée, et de préférence celle raccordée au côté horizontal de l'embouchure, de niveau le plus élevé, les deux parois peuvent être inclinées ou incurvées.

De plus, selon un autre mode de réalisation, l'embouchure peut être de forme générale trapézoïdale dont les bases seront horizontales et raccordées à des parois planes et parallèles, tandis que les côtés (dont au moins l'un pourrait être courbe) seraient raccordés à des parois inclinées ou incurvées. Contrairement aux modes de réalisation mentionnés ci-avant, on comprend que les parois planes et parallèles sont alors raccordées aux côtés horizontaux.

Selon un mode de réalisation, certains au moins des bords de l'embouchure de la cellule sont chanfreinés pour assurer un meilleur écoulement du surplus de produit.

Une cellule selon l'invention possède de multiples applications. Pour mesurer la teneur en humidité des céréales par effet capacitif, une cellule selon l'invention peut avantageusement être utilisée et dans ce cas, les armatures du condensateur de mesure sont, par exemple, constituées par les deux parois planes et parallèles de ladite cellule. De la sorte, on effectue bien une mesure sur tout le produit contenu (ou quasiment tout le produit).

L'invention sera bien comprise et d'autres particularités apparaîtront à la lecture de la description qui va suivre et qui se réfère aux dessins annexés, dans lesquels:
- les figures 1a à 3a montrent schématiquement en élévation une cellule selon l'invention respectivement avant remplissage, en cours de remplisage, et en fin de remplissage.
- Les figures 1b à 3b sont des vues selon les flèches F1, F2 et F3 des figures respectivement 1a à 3a.

Sur les dessins, on peut voir une cellule de mesure 1, destinée à recevoir un produit granuleux ou pulvérulent 2 initialement contenu dans une trémie d'alimentation 3, disposée juste au-dessus de la cellule 1 (si le produit 2 est rendu visible sur les dessins c'est évidemment artificiel pour une meilleure compréhension).

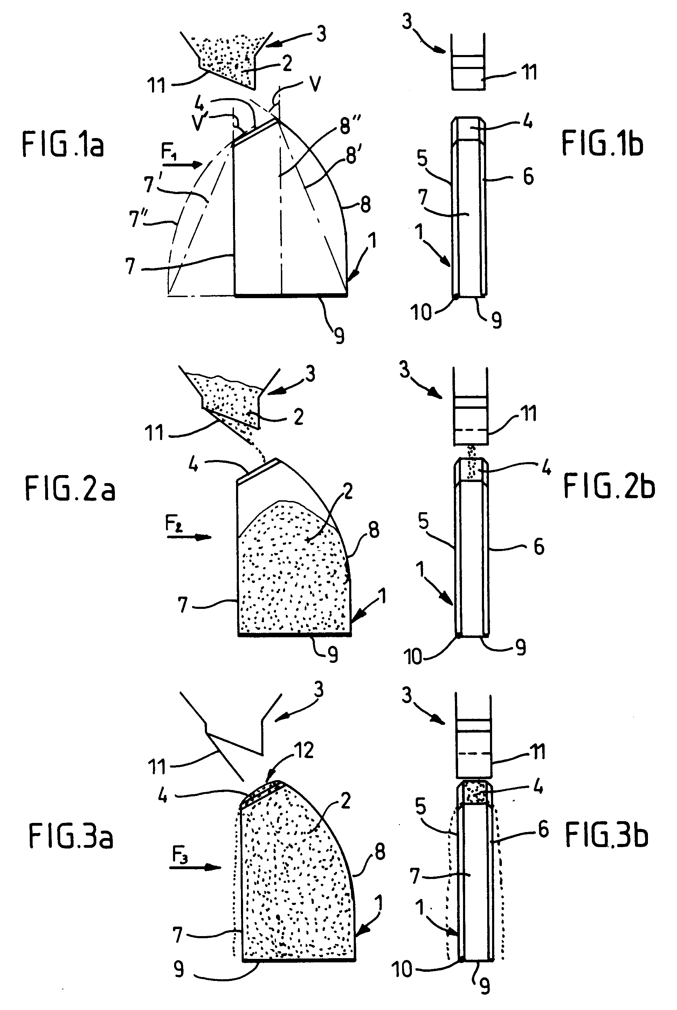

La cellule 1 comporte une embouchure 4, qui est ici rectangulaire (figures 1b, 2b, 3b), et qui est disposée en position de fonctionnement, comme le montrent les dessins selon un plan incliné, c'est-à-dire que ledit plan forme un angle dièdre aigu avec un plan horizontal, les petits côtés étant ici horizontaux tandis que les grands côtés sont inclinés.

Dans le mode de réalisation représenté, on peut en outre constater que l'embouchure 4 prend toute la surface de la partie supérieure de la cellule 1.

La cellule 1 comporte, de plus, deux parois latérales 5 et 6 qui sont planes et parallèles (figures 1b, 2b et 3b) et qui se raccordent sur les deux grands côtés opposés et inclinés de l'embouchure 4. Par ailleurs, des parois 7 et 8 (figures 1a, 2a et 3a) sont raccordées sur les deux autres côtés de l'embouchure, c'est-à-dire sur les petits côtés horizontaux, la paroi 7 étant raccordée sur le côté de niveau le plus bas et la paroi 8 sur le côté de niveau le plus haut.

Les quatre parois 5, 6, 7 et 8 rejoignent, à l'opposé de l'embouchure 4, un fond 9. Le fond 9 de la cellule est, par exemple, constitué par un volet articulé en rotation autour d'un axe 10 (figures 1b, 2b, 3b), afin de permettre le vidage de ladite cellule.

Comme le montrent aussi les dessins, la paroi 7 est verticale tandis que la paroi 8 est incurvée à partir d'une certaine hauteur vers ladite paroi opposée 7.

Cette forme est particulièrement avantageuse, mais non obligatoire.

Sur la figure 1a (avant remplissage), on a représenté en traits mixtes deux autres formes possibles 7′ et 7˝ (respectivement inclinée et incurvée) pour la paroi 7 et deux autres formes 8′ et 8˝ (respectivement inclinée et verticale) pour la paroi 8. Toutes les combinaisons sont évidemment possibles et on peut en imaginer d'autres, surtout si l'embouchure 4 ne prend pas toute la partie supérieure de la cellule 1, contrairement au mode de réalisation représenté.

Il est clair aussi que les petits côtés de l'embouchure pourraient être les côtés inclinés et les grand côtés, les côtés horizontaux, de même que ladite embouchure pourrait être circulaire, elliptique ou autre. De même, comme déjà dit, les parois planes pourraient être raccordées sur deux côtés horizontaux de l'embouchure, une ou des parois inclinées ou incurvées étant raccordées sur des côtés inclinés de ladite embouchure.

Toutefois, l'inventeur a cherché une forme convenable qui, à la fois, facilite le remplissage complet de la cellule, et maximise son volume utile, tout en minimisant le volume du dôme résiduel 12 de produit (figure 3a) dont il sera question de nouveau ci-après.

Sur les dessins, on a représenté une trémie d'alimentation 3, muni d'un volet d'ouverture 11, inclinée de manière classique comme représentée.

Comme le montrent bien les figures, l'inclinaison du volet 11 est opposée à celle de l'embouchure 4, alors que la paroi 8 est incurvée dans le sens de l'écoulement du produit 2 (figure 2a). Toutefois, dans le cas d'un raccordement des parois planes sur les côtés horizontaux de l'embouchure et des parois inclinées ou incurvées sur les côtés inclinés, la deuxième caractéristique précitée disparait, mais la première est conservée, à savoir l'inclinaison du volet 11 opposée à celle de l'embouchure 4.

A la fin du remplissage (figures 3a et 3b), c'est-à-dire jusqu'à ce que le produit 2 déborde, ledit produit forme un léger dôme 12 au-dessus de l'embouchure 4 et on comprend que ce dôme est limité du fait que ladite embouchure est inclinée.

Pour diverses raisons, la pente de cette embouchure est avantageusement inférieure à 45°, par exemple 30°. Dans ce cas, le dôme étant petit grâce à l'écoulement du surplus de produit (figures 3a et 3b), ses variations en volume lors de remplissages successifs sont particulièrement faibles.

Pour un bon remplissage de la cellule, il est en outre avantageux que le plan tangentiel à la paroi incurvée contenant le côté horizontal de l'embouchure auquel ladite paroi incurvée est raccordée, forme avec la verticale un angle suffisamment aigu, par exemple inférieur à 70° et notamment 60° (voir les angles V et V′ de la figure 1a).

Pour assurer un meilleur écoulement du surplus du produit lors du remplissage, certains au moins des bords de l'embouchure 4 sont chanfreinés comme le montrent les figures 1b, 2b et 3b pour les bords inclinés de ladite embouchure.

Comme on l'a déjà dit, une cellule selon l'invention peut être utilisée pour diverses applications et différents capteurs de mesure peuvent être associés à ladite cellule.

Dans le but de constituer une cellule de mesure de la teneur en humidité du produit par effet capacitif (en dehors d'autres mesures tels que le poids spécifique etc.), dans le mode de réalisation décrit, les parois latérales planes 5 et 6 constituent avantageusement les armatures du condensateur de mesure.

## Revendications

1. Cellule de mesure (1) pour produits (2) granuleux ou pulvérulents comportant à sa partie supérieure une embouchure de remplissage (4), caractérisée en ce que le plan de son embouchure de remplissage, en position de fonctionnement, forme un angle dièdre aigu avec un plan horizontal afin de limiter par effet gravitaire l'importance du dôme (12) formé par le produit après remplissage complet de la cellule, la mesure s'effectuant sur la quasi-totalité du produit ainsi contenu.

2. Cellule de mesure selon la revendication 1, caractérisée en ce que l'angle dièdre aigu formé par le plan de son embouchure (4) est inférieur ou égal à 45°.

3. Cellule de mesure selon l'une des revendications 1 et 2, caractérisée en ce que l'embouchure de remplissage (4) de la cellule (1) s'ouvre sur toute la surface de la partie supérieure de ladite cellule.

4. Cellule de mesure selon l'une des revendications 1 à 3, caractérisée en ce que la section horizontale de la cellule en position de fonctionnement est au moins en partie décroissante en partant du fond (9) de ladite cellule (1) vers son embouchure (4).

5. Cellule de mesure selon l'une des revendications 1 à 4, caractérisée en ce qu'elle comporte une embouchure (4) rectangulaire, deux parois latérales (5,6) planes et parallèles raccordées sur les deux côtés opposés et inclinés de l'embouchure (4) et deux autres parois raccordées sur les deux autres côtés (7,8) horizontaux de ladite embouchure, toutes ces parois se prolongeant en outre à l'opposé de l'embouchure jusqu'au fond (9) de la cellule (1).

6. Cellule de mesure selon l'une des revendications 3, 4 et 5, caractérisée en ce qu'au moins l'une (8′,7′) des parois, raccordée sur l'un des côtés horizontaux de l'embouchure, et qui part du fond (9) de la cellule, est au moins en partie inclinée vers la paroi opposée (7,8) qui est, elle, raccordée sur l'autre côté horizontal de l'embouchure.

7. Cellule de mesure selon l'une des revendications 3, 4 et 5, caractérisée en ce qu'au moins l'une (8,7˝) des parois, raccordée sur l'un des côtés horizontaux de l'embouchure, et qui part du fond de la cellule, est au moins en partie incurvée vers la paroi opposée (7,8) qui est, elle, raccordée sur l'autre côté horizontal de l'embouchure.

8. Cellule de mesure selon la revendication 7, caractérisée en ce que le plan tangentiel à la paroi incurvée (8,7˝) contenant le côté horizontal de l'embouchure auquel ladite paroi incurvée est raccordée, forme avec la verticale un angle (V,V′) inférieur à 70°.

9. Cellule de mesure selon l'une des revendications 1 à 8, caractérisée en ce que certains au moins des bords de l'embouchure de la cellule sont chanfreinés pour assurer un meilleur écoulement du surplus de produit.

10. Cellule de mesure selon l'une des revendications 5 à 8, notamment destinée à mesurer la teneur en humidité des céréales par effet capacitif, caractérisée en ce que les armatures du condensateur de mesure sont constituées par les deux parois (5,6) planes et parallèles de ladite cellule (1).

## Claims

1. Measuring cell (1) for granular or powdery products (2) comprising in its upper part a filling mouth (4), characterised in that in the operating position the plane of its filling mouth forms an acute dihedral angle with a horizontal plane in order to limit by gravity the size of the dome (12) formed by the product after complete filling of the cell, the measurement being effected over the quasi-totality of the product thus contained.

2. Measuring cell according to claim 1, characterised in that the acute dihedral angle formed by the plane of its mouth (4) is less than or equal to 45°.

3. Measuring cell according to one of claims 1 and 2, characterised in that the filling mouth (4) of the cell (1) opens over the entire surface of the upper part of said cell.

4. Measuring cell according to one of claims 1 to 3, characterised in that in the operating position the horizontal section of the cell decreases at least in part from the bottom (9) of said cell (1) towards its mouth (4).

5. Measuring cell according to one of claims 1 to 4, characterised in that it comprises a rectangular mouth (4), two flat and parallel side walls (5, 6) connected to the two opposing inclined sides of the mouth (4) and two other walls connected to the two other horizontal sides (7, 8) of said mouth, all these walls additionally extending in the opposite direction from the mouth to the bottom (9) of the cell (1).

6. Measuring cell according to one of claims 3, 4 and 5, characterised in that at least one (8', 7') of the walls, connected to one of the horizontal sides of the mouth, and which runs from the bottom (9) of the cell, is inclined at least in part towards the opposite wall (7, 8) which is itself connected to the other horizontal side of the mouth.

7. Measuring cell according to one of claims 3, 4 and 5, characterised in that at least one (8, 7'') of the walls, connected to one of the horizontal sides of the mouth, and which runs from the bottom of the cell, is curved at least in part towards the opposite wall (7, 8) which is itself connected to the other horizontal side of the mouth.

8. Measuring cell according to claim 7, characterised in that the plane tangential to the curved wall (8, 7'') containing the horizontal side of the mouth to which said curved wall is connected, forms an angle (V, V') of less than 70° with the vertical.

9. Measuring cell according to one of claims 1 to 8, characterised in that at least some of the edges of the mouth of the cell are chamfered to ensure better flow of the surplus product.

10. Measuring cell according to one of claims 5 to 8, designed in particular for measuring the moisture content of cereals by capacitive effect, characterised in that the plates of the measuring condenser are constituted by the two flat and parallel walls (5, 6) of said cell (1).

## Patentansprüche

1. Meßzelle (1) für körnige oder pulverige Erzeugnisse (2), die an ihrem Oberteil eine Einfüllöffnung (4) aufweist, dadurch gekennzeichnet, daß die Ebene ihrer Einfüllöffnung in der Betriebsstellung mit einer horizontalen Ebene einen spitzen Flächenwinkel einschließt, um durch eine Gravitationseinwirkung den Einfluß des vom Erzeugnis nach dem vollständigen Befüllen der Zelle gebildeten Kegels (12) zu begrenzen, so daß die Messung an der Quasi-Gesamtheit des in ihr enthaltenen Erzeugnisses durchgeführt wird.

2. Meßzelle nach Anspruch 1, dadurch gekennzeichnet, daß der durch die Ebene ihrer Einfüllöffnung (4) gebildete spitze Flächenwinkel kleiner oder gleich 45° ist.

3. Meßzelle nach einem der Ansprüche 1 und 2, dadurch gekennzeichnet, daß die Einfüllöffnung (4) der Zelle (1) sich über die ganze Oberfläche des Oberteils der besagten Zelle erstreckt.

4. Meßzelle nach einem der Ansprüche 1 bis 3, dadurch gekennzeichnet, daß der horizontale Querschnitt der Zelle in der Betriebsstellung, ausgehend vom Boden (9) der besagten Zelle (1) zu ihrer Einfüllöffnung (4) hin, mindestens zum Teil abnimmt.

5. Meßzelle nach einem der Ansprüche 1 bis 4, dadurch gekennzeichnet, daß sie eine rechteckige Einfüllöffnung (4) aufweist, sowie zwei ebene, parallele, mit den beiden einander gegenüberliegenden und schräg verlaufenden Seiten der Öffnung (4) verbundene Seitenwände (5, 6) und zwei weitere, mit den beiden anderen, horizontalen Seiten der besagten Öffnung verbundene Seitenwände (7, 8), wobei sich alle diese Wände weiter in der von der Öffnung abgewandten Richtung bis zum Boden (9) der Zelle (1) hin ausdehnen.

6. Meßzelle nach einem der Ansprüche 3, 4 und 5, dadurch gekennzeichnet, daß mindestens eine (8', 7') der mit einer der horizontalen Seiten der Öffnung verbundenen Wände, die vom Boden (9) der Zelle ausgeht, mindestens zum Teil gegen die gegenüberliegende Wand (7, 8) geneigt ausgebildet ist, welche ihrerseits mit der anderen, horizontalen Seite der Öffnung verbunden ist.

7. Meßzelle nach einem der Ansprüche 3, 4 und 5, dadurch gekennzeichnet, daß mindestens eine (8, 7'') der mit einer der horizontalen Seiten der Öffnung verbundenen Wände, die vom Boden der Zelle ausgeht, mindestens zum Teil gegen die gegenüberliegende Wand (7, 8) nach innen gekrümmt ausgebildet ist, welche ihrerseits mit der anderen horizontalen Seite der Öffnung verbunden ist.

8. Meßzelle nach Anspruch 7, dadurch gekennzeichnet, daß die Tangentialebene an die gekrümmte Wand (8, 7''), welche die horizontale Seite der Öffnung enthält, mit der die besagte gekrümmte Wand verbunden ist, mit der Vertikalen einen Winkel (V, V') kleiner als 70° einschließt.

9. Meßzelle nach einem der Ansprüche 1 bis 8, dadurch gekennzeichnet, daß mindestens bestimmte der Ränder der Öffnung der Zelle angefast sind, um ein besseres Abfließen des Überschusses des Erzeugnisses sicherzustellen.

10. Meßzelle nach einem der Ansprüche 5 bis 8, insbesondere bestimmt zur Messung des Feuchtigkeitsgehalts von Getreide durch kapazitiven Effekt, dadurch gekennzeichnet, daß die Beläge des Meßkondensators durch die beiden ebenen und parallelen Seitenwände (5, 6) der besagten Zelle (1) gebildet werden.
